(19)

**Europäisches Patentamt**
**European Patent Office**
**Office européen des brevets**

(11)  **EP 1 358 345 B1**

(12)  **EUROPEAN PATENT SPECIFICATION**

(45) Date of publication and mention
of the grant of the patent:
**21.10.2009  Bulletin 2009/43**

(21) Application number: **01902877.8**

(22) Date of filing: **07.02.2001**

(51) Int Cl.:
*C12Q 1/68* *(2006.01)*    *G01N 27/26* *(2006.01)*

(86) International application number:
**PCT/PL2001/000012**

(87) International publication number:
**WO 2002/063043 (15.08.2002 Gazette 2002/33)**

(54) **MULTITEMPERATURE SINGLE STRAND CONFORMATION POLYMORPHISM (MSSCP)**

MULTITEMPERATURE "SINGLE STRAND CONFORMATION POLYMORPHISM" (MSSCP)

POLYMORPHISME DE CONFORMATION MONOBRIN MULTITEMPERATURE (MSSCP)

(84) Designated Contracting States:
**AT BE CH CY DE DK ES FI FR GB GR IE IT LI LU MC NL PT SE TR**

(43) Date of publication of application:
**05.11.2003  Bulletin 2003/45**

(73) Proprietor: **Kucharczyk, Krzysztof**
**02-495 Warszawa (PL)**

(72) Inventor: **Kucharczyk, Krzysztof**
**02-495 Warszawa (PL)**

(74) Representative: **Friede, Thomas**
**Patent- und Rechtsanwälte**
**Bardehle - Pagenberg - Dost**
**Altenburg - Geissler**
**Galileiplatz 1**
**80679 München (DE)**

(56) References cited:
**WO-A-00/20853**

• **GRACE M ET AL: "Transverse temperature gradient single strand conformation polymorphism analysis for temperature optimization of "Cold"-SSCP mutation detection" NUCLEIC ACIDS RESEARCH, vol. 23, no. 20, 25 October 1995 (1995-10-25), pages 4224-26, XP002180763**

• **CHEN X ET AL: "High resolution SSCP by opimization of the temperature by transverse TGGE" NUCLEIC ACIDS RESEARCH, vol. 23, no. 21, 11 November 1995 (1995-11-11), pages 4524-25, XP002180764**

• **SUGANO K ET AL: "Detection of K-ras and p53 mutations by temperature gradient single-strand conformation polymorphism (TG-SCCP) analysis" PROCEEDINGS OF THE AMERICAN ASSOCIATION FOR CANCER RESEARCH ANNUAL MEETING, vol. 37, March 1996 (1996-03), page 598 XP002180765**

• **RÜBBEN A ET AL : "Evaluation of non-radioactive temperature gradient SSCP analysis and of temperature gradient gel electrophoresis for the detection of HPV 6-variants in condylomata and Buschke-Loewenstein tumours" EUROPEAN JOURNAL OF EPIDERMOLOGY, vol. 11, no. 5, October 1995 (1995-10), pages 501-6, XP001030776**

• **HUUSKO P ET AL: "Germ-line TP53 mutations in Finnish cancer families exhibiting features of the Li-Fraumeni Syndrome and negative for BRCA1 and BRCA2" CANCER GENETICS AND CYTOGENETICS, vol. 112, no. 1, July 1999 (1999-07), pages 9-14, XP001030775**

Remarks:
The file contains technical information submitted after the application was filed and not included in this specification

**Description**

FIELD OF THE INVENTION:

Methods of the present invention relate to a means for rapid detection and characterization of nucleic acid sequences and sequence variability. The present invention relates to means for changing the separation mobility of single strand nucleic acids in a temperature dependent manner. Changes of the separation mobility are used for detection of known and unknown single base changes in nucleic acids.

BACKGROUND OF THE INVENTION

Genetic variability observed in between a specimen of any species is the result of polymorphism developed during the biological evolution and spontaneous mutations accumulated in during the life span of individuals.

Establishing the correlation between the genetic variations, the environment and observed phenotype of an organism (or a population) could provide a vital information for understanding the basic mechanisms of live.

One of the most often observed polymorphisms is a single base pair distinction in the sequence of a nucleic acid strand, compared to the most prevalently found (wild type) nucleic acid strand, called Single Nucleotide Polymorphism (SNP). When SNP occurs in the gene coding for a structural protein or it's regulating region, such genetic polymorphism might influenced the functionality of the coded protein, cells and the whole organism phenotype.

In most cases, in higher organisms the observed phenotype is the result of interacting of several genes products. Since the compensation/adjustment mechanism existing on the protein level could influence the final phenotype, the polymorphism pattern and mutation frequency correlating to any phenotype might be only slightly different in an affected group compared to the control ones. To establish such a correlation, genome-population-wide association studies are usually required.

Since benefits of such studies could significantly improve human life, by e.g. introducing DNA based diagnostics and pharmacogenomics into hospital routine, establishing a reliable methodology of DNA mutation and polymorphism analysis on the genome-population-wide scale is strongly needed. Despite the fact that sequencing of DNA becomes more and more a routine methodology, none of the other actually existing technical methods for genetic sequence variation detection could be applied for e.g. human genome-population-wide genetic surveys in acceptable time and cost scale.

At present, at least three steps could be distinguished at the mutation/SNP detection process. At the first step a decision has to be made which regions/genes of the genome have to be analyzed. In the second stage, a screening for the presence of mutations/SNP in the selected genes/regions has to be conducted and in the last stage sequencing of selected samples might be required.

The mutation/SNPs detection process depends on the amount of the target NA in the analyzed sample. Several standard methods could be used for nucleic acids purification from the starting material, also wide selection of commercial kits are available for nucleic acids purification.

When isolated amount of the target nucleic acids is sufficient for a specific detection, the so called "direct mutation detection" methods could be applied

Methods for "direct detection" of specific sequences in nucleic acids are mostly based on the NA/NA hybridization (e.g. Branched DNA method bDNA - Urdea et al.., Gene 61:253-264 (1987) or protein/NA interaction (Restriction Fragment Length Polymorphism - RFLP).

However when the amount of the target nucleic acids in the analyzed sample is too low for its direct analysis, an amplification step of selected nucleic acids fragments is necessary. The most popular method used for the amplification of the target nucleic acids fragment is Polymerase Chain Reaction on (PCR) as described in U.S. Pat. Nos. 4,683,195 and 4,683,202 to Mullis et al.. The process comprises treating separate complementary strands of the nucleic acid with a molar excess of two oligonucleotide primers, extending the primers with the means of thermo stabile DNA polymerase to form complementary primer extension products, which act as templates for synthesizing the desired nucleic acid sequence, and detecting the sequence so amplified. The steps of the reaction may be carried out stepwise, or simultaneously, and can be repeated as often as desired.

Among another methods which might be used for nucleic acids amplification are:

- Ligase Chain Reaction (LCR or LAR) - described by Barany, Proc.Natl.Acad.Sci., 88:189 (1991). The Amplification of the target NA by the LCR method is based on the unique hybridization of four oligonucleotides to the target NA and cyclic ligation pairs of them. The possibility of unspecific hybridization which could lead to a target independent background signal and the fact that method could be applied for detection of known genetic variations, are limiting the use of the LCR method.
- Self-Sustained Synthetic Reaction (3SR/NASBA) - described by Guatelli et al., Proc.Natl,Acad. Sci., 87:1874-1878 (1990) is a transcription based nucleic acids amplification method that can exponentially amplify 200-300 base pairs long RNA sequences at uniform temperature.

Having the target nucleic acids amplified, several methods could be used for subsequent mutation and polymorphism detection and characterization. All that methods might be divided into two groups:

- biological or specific - like RFLP, hybridization, ASO PCR, pyrosequencing, etc.
- physical or scanning - like: SSCP, DGGE, DHPLC, cleavage based methods, etc.

The comprehensive review of techniques used for SNP/mutation detection was presented in the Electrophoresis No. 6/99, vol.20 or US5719028.

The biological (specific) methods are based on the recognition of a specific nucleic acids sequence - therefore they are primarily suitable for the detection of a known SNP/mutations. In contrast, the second group of methods requires no prior knowledge of investigated sequences and are used for detection and/or identification of any SNP/point mutations. This could be used for e.g. screening of a highly variable genetic regions. Example of the biological methods for genetic diversity detection could be competitive PCR, described in U.S, Patent No. 5,582,989. In that method two different sets of primer pairs are used to amplify a target nucleic acid sequence. According to this method, one set of primers recognizes the wild type sequence and the other set, the selected point mutation. Another example is a method used for detection of the specific SNPs presence in the target nucleic acids based on the Allele Specific Amplification (ASO) described by Shuber (1997) US Pat No. 5,633,134. Unfortunately, PCR reaction could generate false results, because of amplification of nucleic acids sequences to which the primers are not perfectly complementary. Accordingly, depending on the reaction condition (temperature, ionic strength) either set of the competitive primers could prime elongation of either the wild type or the mutant sequence.

Another group of techniques developed for SNP/mutation detection and analysis are based on the hybridization properties of the NA (biochips), see, e.g. Sapolsky et al. (1999) US 5858659; Nerenberger, M, et al (2000) WO 0061805; Arnold, L., et al. (2000) WO 0050869. Also, the mentioned before RFLP method could be used for detection of genetic variability in the amplified nucleic acids fragments.

However, with any of the techniques described above the nature of the suspected genetic variability must be known in advance of the test. Which makes them inapplicable when one needs to detect the presence of a mutation/polymorphism of an unknown character and position.

Only the second group of methods (physico-chemical) are capable of detecting either known, or unknown mutations, and polymorphisms in any selected genome of interest.

One of these methods - Denaturing Gradient Gel Electrophoresis (DGGE), is based on the changes of the electrophoretic mobility in denaturing conditions of the analyzed NA/NA hybrid. In that method, genetic variants can be distinguished, based on the differences in melting properties ofNA homoduplexes versus heteroduplexes, differing in a single nucleotide, which results in changes in their electrophoretic motilities. To increase the number of mutations that can be recognized by DGGE, nucleic acids fragments amplified by PCR reaction are "clamped" at one end by a long stretch of G-C base pairs (30-80) to avoid complete dissociation of the strands while allowing for complete denaturation of the sequence of interest (Abrams et al., Genomics 7:463-475 [1990], Sheffield et al., Proc. Natl. Acad. Sci., 86:232-236 [1989]; and Lerman and Silverstein, Meth. Enzymol., 155:482-501 [1987]). To increase the SNP/mutation detection rate the temperature gradient have been introduced during the separation of amplified NA target by Wartell et al., Nucl. Acids Res., 18:2699-2701 [1990].

Some limitations of that method arise from the fact that the denaturing conditions (temperature and urea concentration) depend on the analyzed sequence, so there is a need for optimization of the denaturing conditions for each target sequence. The final reproducibility of the method depends on the accurate gradient gel preparation and precise gel temperature control. The extra expense caused by the synthesis of the GC clamping tail for each sequence to be tested and quite long time of analysis per sample, are also a major consideration.

One of the most widely used physico-chemical method for SNPs/mutation detection and identification is Single Strand Conformation Polymorphism (SSCP). In the SSCP analysis, single base differences in NA fragments are recognized on the basis of difference in their separation mobility during their separation under the native conditions (Orita, et al., Genomics 5:874-879, (1989). In native conditions the single stranded NA fragments adopt secondary structure according to their sequence and actual physical conditions. Since the electrophoretic mobility of a single strand nucleic acids molecule depends on its net electric charge and 3-D conformation, modification of any single nucleotide in the DNA fragment can result in its different 3-D conformation and thus influence the separation mobility. Some of the single nucleotide modification could, however, result in different 3-D conformation only in particular physico-chemical conditions, of which the most important are the ionic strength, pH and temperature. The number and energetic stability of 2-D conformers of any single strand nucleic acids molecule in the function of the temperature and ionic strength could be calculated, e.g. on the basis of the nearest-neighbor thermodynamics algorithm which is available on line at http://bioinfo.math.rpi.edu/~mfold/dna/.

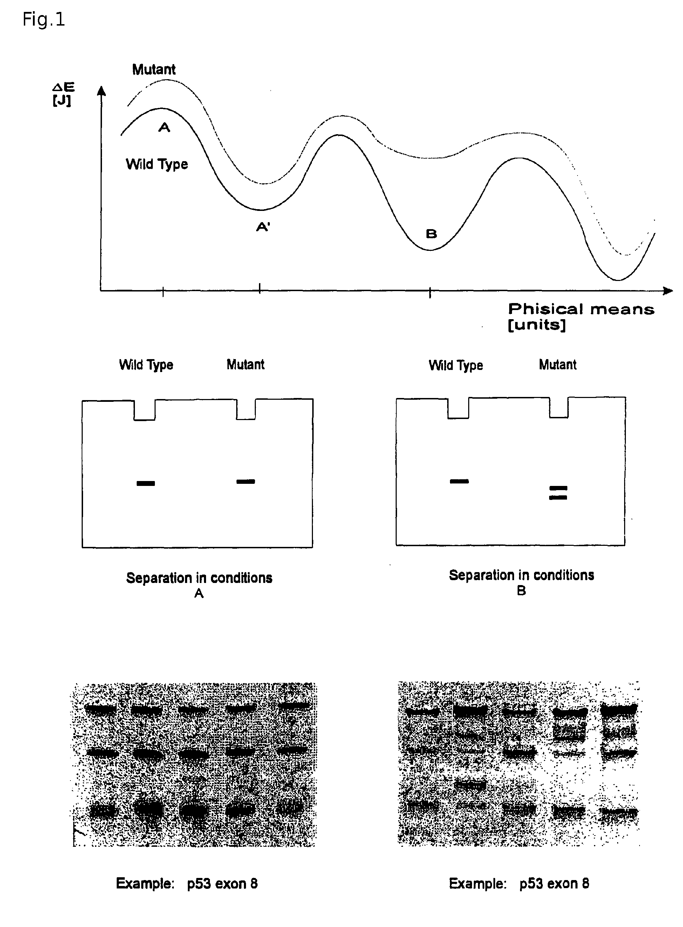

However only in optimal conditions, marked as B on Fig. 1 differences at the separation patterns in between the wt and the mutant nucleic acids was noted. In conditions marked on Fig. 1A, the difference in the spatial conformation of the wt and mutant nucleic acids do not influence the separation mobility to such extent that measurement of that would be possible, so no difference in separation pattern of the two analyzed single strand nucleic acids was observed.

On Fig. 1 are presented two hypothetical nucleic acids molecule wild type sequence with a three different local energetic minima - stabile conformers, and a single base different mutant which possess only two stabile conformers (Fig. 1A section) in the same physical conditions. Separation of such two single strand nucleic acids molecules in native conditions might result in two different separation pattern if conditions of the separation are optimal to express the spatial conformation difference. In constant separation medium composition the biggest influence on the separation profile would possess the separation medium temperature.

The example of the gel temperature influence on the detection of a single nucleotide difference by the SSCP method was shown on Fig. 1C, That same set of five single strand nucleic acids samples from the exon 8 of human p53 gene were loaded on two identical gels and electrophoreses in that same conditions but temperature. On the Fig. 1E and F, the gel temperature was set at 13° C and 23° C, respectively. The influence of the gel temperature on the electrophoretic separation of single strand nucleic acids different in between each other for single base pair was clearly presented.

These findings has been supported by the fact that 2-D structures generated on the basis of the nearest-neighbor thermodynamics algorithm [SantaLucia, Jr., Proc. Natl. Acad. Sci. USA, Vol.95, February 1998, 1460-1465] used by the software which is available at http://bioinfo.math.rpi.edu/~mfold/dna/ have shown significantly different and energetic stable conformers when the temperature was changed for a about 6° C. That supports the idea that any ss DNA molecule could have an individual/optimal temperature for SSCP analysis, which could depend on a G+C content [Kiyama, M., Fujita, T., Biotechniques 21:710-716, 1996]. Nonetheless, changes in the A+T bass's composition are also very easily detected by the SSCP method [Collins, A., Lonjjou, C. and Morton, N., Proc.Natl.Acad. Sci. USA 1999, 96, 15173-15177]. The influence of the gel temperature on the mutation detection in exon 8 of p53 gene, described above, suggests that part of SSCP false negative results might be caused by the inadequate gel temperature control rather than by the method itself. Also we suggest that while comparing the SSCP results from different laboratories, at least the simplified version of the heat transfer model from the gel to the outside have to be applied (equation 1 from Example 1) to estimate the real gel temperature in each experiment. Such gel temperature adjustments should be considered especially when performing the SSCP analysis in slab or CE electrophoresis equipment with an air, solid heat exchange module or with a long reaction time cooling systems.

In spite of the simplicity in performing and relatively high sensitivity (above 90 %) some limitations of the SSCP method have been reported. The most important was the difficulties in obtaining the consistent results and the lack of the theory, which would help to predict the optimal separation conditions (ionic strength, pH, temperature) for particular N.A fragment and lowered them 100 % mutation detection rate. The observed variability of the SSCP according to many authors, depends on the inconsistency of the separation conditions and on the mutation location within the analyzed NA fragment (Glavac and Dean, 1993, Hayashi and Yandell, 1993, Liu and Sommer, 1944). To lower the temperature influence on the e.g. electrophoretic separation, low voltage (power) was applied, however that resulted in very long time of separation - up to 12-14 hours. Sensitivity of the SSCP method could be increased to close to 100 % by applying at least two different separations conditions for the analysis of that same target nucleic acids. The separation conditions which are most influential on the separation mobility of single strand nucleic acids are type of porous support, chemical composition of the separation buffer and temperature as it was described by Liu et. al by WO0020853. However the time and cost of such approach rise proportional to the number of additional separations applied to analyze that same set of samples and would be quite difficult to use in routine diagnostics.

Genetic variability could be, also determined based on the special mass-spectroscopy-analysis of the target NA by Monforte (1998) WO98/12355, Turano et al. (1998) WO98/14616 and Ross et al. (1997) Anal Chem.15, 4197-202; Actually that method requires quite expensive and specialized equipment, which is the major consideration.

Grace et al., Nucl. Acids Res. 23 (1995), 4224 to 4226 describe a transverse temperature-gradient single-strand conformation polymorphism analysis for temperature optimization of Cold-SSCP mutation detection.

Chen et al., Nucl. Acids Res. 23 (1995), 4524 to 4524 describe a high resolution SSCP by optimization of the temperature by transverse temperature-gradient gel electrophoresis.

Rübben et al., Eur, J. Epidemiol. 11 (1995), 301 to 306 describe a non-radioactive temperature gradient SSCP analysis and temperature-gradient gel electrophoresis fort he detection of HPV6-variants.

Summarizing, there is a strong need for an analytical tool that would allow for a reliable, cost and time effective and close to 100 % detection of nucleic acids genetic variability. To become a useful diagnostic or technological tool, it should be ideally operating in full automatic mode with several samples analyzed in that same time, Such methods would allow for more widespread diagnostic screening of human of predispositions for several life threatenings diseases than is currently possible and could result in changing the health care paradigm from diagnoses and treatment to health prevention.

SUMMARY OF THE INVENTION

The present invention relates to a means for changing the native separation mobility of single strand nucleic acids by changes of the physical parameters of the separation conditions. According to the present invention, the means for changing the separation mobility are at least one change of temperature during the native separation of single strand nucleic acids. Changes of the separation mobility of an single strand nucleic acids are used for detection of known and unknown single base changes in nucleic acids for among other uses for research and diagnostics purposes.
The present invention is based on the discovery that changes of the temperature during the native separation of single strand nucleic acids increase differentiation of analyzed molecules based on the difference of the separation mobility of analyzed single strand nucleic acids molecules.

Where the separation mobility of single strand nucleic acids during the native separation is altered, it is not intended that the invention be limited by the means by which the separation mobility is altered. According to the present invention the means is temperature. The change of means may manifest itself in a change of secondary and tertiary conformation of target single strand nucleic acids, as was schematically presented on Fig, 1.

The present invention contemplates means that change the single strand nucleic acids separation mobility during the separation by temperature. Temperature is contemplated as particularly useful as it could be fast and in repeated manner changed during the separation and because the influence of the temperature on the secondary and tertiary single strand nucleic acids structure is significant.

The present invention relates to means for changing the separation mobility of an single strand nucleic acid in a sequence dependent manner. The changes of the separation mobility is used to screen for known and unknown mutations, including single base changes in nucleic acids.

The present invention provides a method as defined in claim 1. Further embodiments are apparent from the dependent claims.

In one embodiment, the present invention contemplates a method for detecting conformation changes in single strand nucleic acid during their separation comprising: a) providing single strand nucleic acid; c) separating the said single strand NA under such conditions that said single strand nucleic acids could form one or more secondary and/or tertiary structures so as to generate a separation pattern of said molecules. By detecting the changes of secondary and tertiary structure, the method of the present invention indirectly detects sequences. The method further comprises step f) comparing said pattern of separated said target single strand nucleic acids with the pattern of a second single strand NA. In such a case the sequence of the second target single strand nucleic acids may be related but different (e.g. a wild type control for a mutant sequence).
In one embodiment, the target single strand nucleic acid contains a fluorescent label and the detection of step e) comprises detection of the said fluorescent labeled fragments.

It is not intended that the invention be limited by either the nature of the separation medium used for nucleic acids separation nor the physical force used for nucleic acids movement trough the medium.
The present invention contemplates different separation conditions (like ionic strength, pH, temperature, viscosity, e.g.), as well as the type and construction of the equipment used for native separation of single strand nucleic acids conformers.

It is not intended that the invention be limited by the nature of the nucleic acid. In the above-described embodiments, the nucleic acid target may be single-stranded DNA, double-stranded DNA, or RNA.

DESCRIPTION OF DRAWINGS

Fig. 1 provides a schematic of one embodiment of the detection method of the present invention and gel temperature influence on the exon 8 human p53 gene mutation detection by the SSCP method.

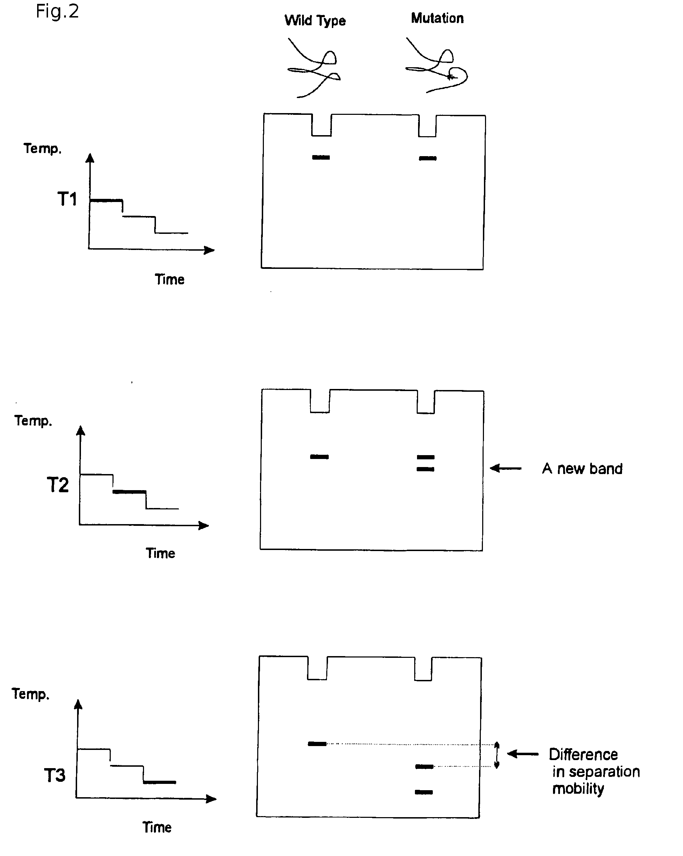

Fig. 2 provides a schematic of one embodiment of the detection method of the present invention, demonstrating the influence of changes of temperature during the native separation of two different single strand NA conformers on their electrophoretic mobility.

Fig. 3 illustrates the influence of the gel temperature modifications during native electrophoresis on the electrophoretic mobility of ssDNA fragments from exon 7 of human PAH gene,

DESCRIPTION OF THE INVENTION

The present invention relates to a means for changing the separation mobility of single strand nucleic acids by changing the temperature during the native separation of the said single strand nucleic acids.

The observation that the changes of a temperature during the native separation of single strand nucleic acids ingresses the differentiation of the separation pattern form the basis of the novel method of detecting specific nucleic acids sequences.

In accordance with the present invention, a "differentiating pattern" might be obtained by the application of these temperature changes during the separation singly or in combination with any changes of any other physical parameters.

The present invention provides a method for differentiations of single strand nucleic acids molecules with putatively different sequences.

The instant invention is to work with any nucleic acid separating equipment or any other sequencing environment involving nucleotide sequence variation determination or detection.

Changes of the separation mobility of an single strand nucleic acids are used for detection of known and unknown single base changes in nucleic acids for among other uses for research and diagnostics purposes.

To facilitate understanding of the invention, a some of terms used here are defined below:

| | |
|---|---|
| NA | Nucleic Acid |
| ss NA | single strand NA |
| "gene" | refers to a DNA fragment that contains control and coding sequences necessary for the synthesis of a polypeptide, or its precursor. |
| "wild-type" | refers to a gene sequence, which is the most common in nature, when it is the structural gene that is usually coding the functional protein. |
| "mutant" | refers to a gene which displays altered sequence when compared to the wild- type gene. The protein coded by mutant gene could have altered characteristics when compared to the wild-type gene product. |
| "sequence variation" | as used herein refers to differences in nucleic acid sequence between two nucleic acid fragments. The examples of sequence variation, includes, but are not limited to, a single base substitutions and/or deletions, or insertions of one or more nucleotides. |
| "native condition" | conditions in which ss NA would adopt is spatial conformation resulted from intermolecular interaction of said molecule atoms, Usually it could be defined by the most critical physical parameters, but is not limited to, like: temperature from 0-50° C, ionic strength from 0-1 M KCl, 6 < pH < 9 |
| "3-D, 2-D" | is a short version of: three-dimensional, two dimensional spatial conformation. |
| "conformer" | refers to a particular 3-D or 2-D spatial conformation of ss NA molecule. It is known that any ss NA molecule could adopt different 3-D conformation depending on the NA sequence and the physical conditions. Stability and 2- D conformations of ss NA molecule could be estimated based on the e.g. nearest-neighbor thermodynamics algorithm [SantaLucia, Jr., Proc. Natl. Acad. Sci. USA, Vol.95, February 1998, 1460-1465] model. It could be seen from that model that for any single ss NA molecule several 2-D conformations might be achieved. The biggest influence on the 2-D conformation of given ss NA molecule possess temperature, ionic strength and pH. It could be also seen from the nearest-neighbor thermodynamics algorithm model that the optimal range of temperatures which could be used for 2-D conformation changes is: 0 -50° C in native condition. |
| "mobility pattern" | as used herein refers to spatial or time distribution of a given ss NA conformers as the result of their separation. Conformers are generated from any nucleic target without reference to a wild type or other control. The invention contemplates the use of the method for both identification based on the "mobility pattern" of any nucleic acids without reference to a control and identification of mutant forms of nucleic acid by comparison of the mutant form with a wild-type or known mutant control |
| "oligonucleotide" | is defined, as a molecule comprised at least two deoxyribonucleotides or ribonucleotides, in practice usually more than ten. The exact size will depend on many factors, which in mm depends on the ultimate function or use of the oligonucleotide. |
| "primer" | refers to an oligonucleotide which is capable of acting as a point of initiation of NA synthesis when placed under conditions in which primer extension is initiated. |
| "label" | refers to any atom or molecule which can be used to provide a detectable (preferably quantifiable) signal, and which can be attached to a nucleic acid. Labels may provide signals detectable by any of the physical measures, like: electromagnetic waves, fluorescence, radioactivity, X-ray diffraction or absorption, magnetism, and the like. |
| "optimal separation conditions" | refers to a set of conditions that yields the most distant Separation of bands present in a one sample, with the most even signal intensity between the bands. The examples |

of modified conditions, includes, but are not limited to, temperature, ionic strength, pH etc.

Depending on the conditions and primary sequence nucleic acid assume secondary structure. Nucleic acid with at least one single base difference would assume different 2-D and/or 3-D structures, which results in their different separation mobility. Recording the separation of said different nucleic acids in time or space, as was schematic represented on Fig.1, results in two different separation patterns. Ideally any single base change would affect separation mobility pattern of given single strand nucleic acids.

According to the method of the present invention for separation are used molecules which possess the 3-D structures formed after renaturation of complete denatured target nucleic acids. The denaturation of nucleic acids may be achieved by treating them with physical, chemical or enzymatic means which disrupt the secondary nucleic acids structure like, low (<3) or high pH (>10), high temperature, low salt concentrations or chemicals, like e.g., urea, formamide or proteins (e.g., helicases) or combination of them. The most effective and simple for use seems to be combinations of high temperature (about 100 °C) in presence of, e.g. formamide, or urea. Lowering of the temperature, addition of salt, neutralization of the pH, withdrawal of the chemicals or proteins achieve folding or renaturation of the nucleic acid. When denaturation means are removed, a collection of molecules with unique 3-D structure dependent on its sequence and physical conditions is received. These conformers constitute a characteristic mark of the nucleic acid, which could be detected by separation of these conformers on a porous media in optimal conditions.

Both, changes in the sequence of a nucleic acid and physical parameters of medium, alter the spatial conformation of nucleic acids, which result in different separation pattern reflecting the difference in the sequence of the analyzed molecule or changes of the analysis conditions. The fact that the environment parameter could so strongly influence the separation pattern (that could be the causes of reported in the art low reliability of the SSCP analysis) formed a basis to increase the probability of receiving two different separation patterns, when analyzing two nucleic acids with a very similar sequence. According to the method of the present invention, conditions during the native separation of nucleic acids conformers are changed at least one time. That increases the probability of retaining another 2-D and/or 3-D structure by analyzed ss nucleic acid during the analysis and increase probability of receiving two different separations pattern.

According to the present invention, changing the medium temperature during the separation increases the probability of retaining different spatial conformation by analyzed nucleic acid. Changes of the temperature during separation can be used to detect single base changes in nucleic acid molecule in convenient and fast manner. The method of the invention is termed "Multitemperature Single Stand Conformation Polymorphism" abbreviation "MSSCP".

Target nucleic acid that may be analyzed by the MSSCP method includes both RNA and DNA. Such nucleic acid target may all be obtained using standard molecular biological techniques. For example, target NA may be isolated from a tissue sample or culture, cells, bacteria or viruses, may be transcribed in vitro from a DNA template, or may be chemically synthesized. Furthermore, substrates may be isolated from an organism, either as genomic material or as a plasmid or extra chromosomal DNA, or it may be a fragment of such material generated by treatment with a restriction endonuclease or other cleavage agents or it may be synthetic,

Target nucleic acids may also be produced by amplification using the PCR. When the target is to be a single-stranded molecule, the target may be produced using the PCR with preferential amplification of one strand (asymmetric PCR). Single-stranded target may also be generated in other ways known from the art like by digestion of one strand of double-stranded molecule with exonuclease,

The target nucleic acids may contain a label to aid in their detection following the separation. The label may be a radioisotope placed at either the 5' or 3' end of the nucleic acid. Also fluorophore label could be used which can be detected directly, or any reactive group which permits specific recognition by a secondary agent. For example, biotinylated nucleic acids may be detected by probing with a streptavidin molecule, which is coupled to an indicator (e.g., enzyme, or a fluorophore).

In a preferred embodiment, the unlabeled nucleic acid is visualized by staining with sliver ions or available single strand nucleic acids commercial stains. Also when sufficient quantities of DNA are available for the analysis, direct fluorescence of nucleic acid fragments would allow for parallel analysis of several samples. Such approach would be especially useful for automated comparisons of e.g. wild type and mutant forms of a gene separated on that same gel.

The spatial conformer of the target nucleic acid with different 3-D conformation might be analyzed and resolved by several methods including electrophoresis, chromatography, and fluorescence polarization. The invention is illustrated using electrophoretic separation. However, it is noted that the separation of the target conformers is not limited to electrophoresis. Separation of single strand nucleic acid conformers in electric field is described to illustrate the method of the invention since the electrophoresis is one of the most popular methods used for biological molecules separation.

The MSSCP reaction is useful to rapid and cost effective screen for single base sequence differences between nucleic acid molecules. To optimize the MSSCP reaction for any desired nucleic acid target (e.g., a wild-type nucleic acid and one or more mutant forms of the wild-type nucleic acid), it could be convenient to use the wild-type form and one single base pair mutant to determine the best MSSCP conditions (temperature steps and salt concentration) which allow the target molecules to form a two most clearly different separation patterns.

Likewise, other factors affecting nucleic acid structures, such as, formamide, urea or extremes in pH may be used. The initial test typically will comprise two separations of 3-D conformers at four temperatures during one run: 45 °C, 30 °C, 15 °C and 5°C. Also the salt concentrations might be selected, e.g. 5 mM or 35 mM. It is not intended that the salt utilized limit the present invention. The salt utilized may be chosen from potassium chloride, sodium chloride, etc.

To date, each single nucleotide difference in analyzed nucleic acid target analyzed by the MSSCP method has produced a different and reproducible pattern of fragments. The short time of electrophoresis (3 min /per sample), close to 100 % sensitivity and specificity and reproducibility thanks to precise separation medium temperature control method make this method of analysis very suitable for the rapid screening of genetic variability in genome-population-wide surveys.

Especially for purposes like in cancer diagnostics, tissue typing, genetic identity, bacterial typing, mutant screening in genetic crosses, etc. One distinct benefit of using the MSSCP method is that the pattern of separated conformers in particular combination of physical conditions constitutes a characteristic fingerprint, so a potential mutant can be compared to previously characterized mutants without sequencing. Also, the band which express different mobility them the wild type could be used as a matrix for subsequent sequencing reaction without the needs for cloning procedure. That would significantly improve the WO 02/063043 PCT/PL01/00012 sequencing reaction quality, since only one single strand NA is used for reamplification reaction. Thus that approach could very quickly lead to discovery of new SNP or single point mutations.

EXPERIMENTAL

The following example serve to illustrate certain preferred embodiments and aspects of the present invention and is not to be construed as limiting the scope thereof.

In the part which follows, the following abbreviations apply: amplicons ( amplified fragments of DNA produce by the PCR reaction); vol (volume); w/v (weight to volume); v/v (volume to volume); DNA (deoxyribonucleic acid); ml (milliliters); M (molar); mM (milliMolar); EDTA (ethylene diamine tetra-acetic acid); SDS (sodium dodecyl sulfate); Tris (tris(hydroxymethyl)-aminomethane); TBE (Tris-Borate-EDTA, i.e., Tris buffer titrated with boric acid and containing EDTA); PBS (phosphate buffered saline); PAGE (polyacrylamide gel electrophoresis); Perkin Elmer (Norwalk, Con.); Promega Corp. (Madison, Wis.); Kucharczyk Inc. (Warszawa, Poland)

Example 1

1) Construction of DNA Pointer System.

In order to control the gel temperature during electrophoresis, the dedicated DNA Pointer System has been built up. This System consists of three modules:

I - the slab gel electrophoresis chamber with two cooling chambers, 2 - heat exchange module - based on thermoelectric Peltier cells and an efficient circulation system, 3 - control unit - a personal computer with dedicated software that allows to control and program the gel temperature profile during the electrophoresis, considering the amount of heat generated by the current flow through the gel.

The basic DNA Pointer System parameters are as follows: Gel temperature range: 2°-65° C, gel temperature accuracy: 0,1 °C, gel size (W x H): standard model - 150 x 150 mm. The software algorithm controlling the gel temperature is based on the following equation describing heat transfer:

$$\Delta T \approx Q_o/(2\ A)\ *\ (\tfrac{1}{4}\ \Delta x1\ /\ \lambda1 + \Delta x2\ /\ \lambda2\ +\ 1\ /\ \lambda3) \qquad (1)$$

Where:

$\Delta T$ - temperature difference between the cooling medium and the gel
$Q_o$ - heat generated inside the gel
A - surface of the heat conductivity (gel glass cooling surface)
$\Delta x1$ - thickness of the gel
$\lambda1$ - thermal conductivity of the gel

Δx2- thickness of the glass plates

λ2 - thermal conductivity of the glass plates

λ3 - convection heat transfer coefficient between glass plate and coolant

Example 2

The influence of the gel temperature changes during the native separation of single strand nucleic acid conformers on the detection of a single nucleotide difference in the exon 7 of human PAH gene.

1) Amplification of exon 7 Phenylalanine Hydroxylase human gene.

DNA preparation to amplify fragments from exon 7 of the human PAH gene was done by standard procedure. Exon 7 was amplified by PCR reaction using primers AP287 - (TGCCTCTGACTCAGTGGTGAT) and AP423 - (CCCAAACCTCATTCTTGCAGCA).
PCR reactions contained 100 ng genomic DNA as a template, 50 mM Tris-HCl, 50 mM KCl, 2 mM MgCl$_2$, 2 μM of each primer, 0,2 mM of dNTPs, and 2U/100 μl of Taq polymerase (Kucharczyk Inc., Warsaw). PCR was performed in 25 μl, cycling conditions were as follows: 94°C for 5 min followed by 31 cycles of 94°C, for 30 sec, 58°C for 30 sec, 72°C for 45 sec with the final extension at 72°C for 5 minutes.

2) MSSCP and SSCP analysis of amplicons from the exon 7 human PAH gene.

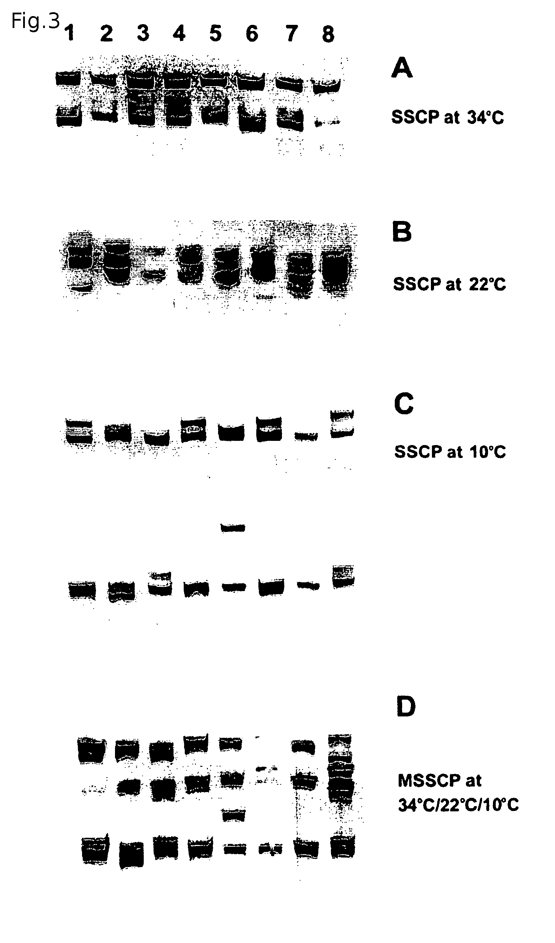

1 μl of PCR product from p.1) was added to 5 μl of 7 M urea, 2 μl of gel-loading buffer (0,25% (w/v) bromophenol blue, 0,25% (w/v) xylene cyanol FF, 30 % (v/v) glycerol in water). 4 μl of H$_2$O was added to total volume of 12 μl. The mixture was heated to 94°C for 2 minutes and then cooled on ice. The cooled mixture was immediately loaded onto 9 % (w/v) native polyacrylamide gel (9 % acrylamide: bisacrylamide - 29 : 1, containing 5% (v/v) glycerol). Electrophoresis was carried out in 1 x TBE in DNA Pointer Mutation Detection System (Kucharczyk Inc., Warsaw) with 40 W constant power. First three separate SSCP electrophoresis were performed at following constant gel temperatures: 34° C, 22°C and 10°C and each lasted for 1000 Vh. After electrophoresis the ss NA bands resolved in all gels were visualized by silver staining using the Kucharczyk Inc., Warsaw, Silver Stain kit, dried and scanned. The results are shown in Fig 3 A, B and C, where gel temperatures were: 34°C, 22 °C and 10 °C respectively.
In Fig 3, the lane marked with numbers from 1 to 6 and number 8 contain ss NA from eight different single point mutants from exon 7 human PAH gene and in the line number 7 wild type version.
Selection of the optimal gel temperature for SSCP analysis is at present based mainly on the empirical data. Fig 3 It was shown that by changing the gel temperature during the native separation of target nucleic acid we increase the electrophoretic pattern differentiation.
That was shown when analyzing eight different alleles of the exon 7 of human PAH gene. Fig. 3 A, B, C shows that while the pattern of resolved ss DNA conformers generated from each mutant changes as the temperature of the separation is decreasing, only 5, 5 and 6 distinct patterns are generated during the SSCP electrophoresis at constant gel temperatures 34° C, 22° C and 10° C, respectively. And not all analyzed samples could be distinguished from each other and from wild type.
However, during single MSSCP electrophoresis, when the gel temperature was changed from 34° C to 22° C and next to 10° C, for 333 Vh during electrophoresis at each temperature, distinct pattern are generated from each mutant and the wild type as shown at Fig. 3 D.
All analyzed samples have clearly different electrophoretic patterns and are easily distinguished from each other and from the wild type. Total time of the MSSCP electrophoresis was about 65 minutes (1000 Vh). After electrophoresis gels were silver stained during 30 minutes using Silver Stain Kit (Kucharczyk Inc., Warsaw) and afterwards dried in Dryout gel drying unit (Kucharczyk Inc., Warsaw) and then scanned and analyzed using Gelscan software (Kucharczyk Inc., Warsaw) These data suggest that not only selected temperatures are the sufficient conditions to detect all sequence variants in single strand DNA fragments, but also the fact of changing the gel temperature during the electrophoresis increase the electrophoretic difference in between analyzed samples.
With the help of the MSSCP technology we have analyzed other SNPs and point mutations in the human genes, like APOE, APOB, LHR and in each case a higher mutation detection rate or a clearer allele differentiation in comparison to the classical SSCP method was received (manuscripts in preparation). Based on this accumulated evidence we conclude that the sensitivity of the MSSCP method is significantly higher than published SSCP mutation detection rate.
Also, the total time of analysis and cost of chemicals used in our experiments, which goes to about 4 minutes and below 0.2 USD per sample respectively are significant improvements over the classical SSCP method. Additionally, because of the low volume of PCR reaction with non-labeled primer used for DNA fragment amplification, the cost of the PCR reaction and so the overall cost of the SNP/mutation screening approach was lowered. Combining the MSSCP analysis with a fast and effective electro-elution method of selected ssDNA molecule from the PA gel for subsequent sequencing reaction (manuscript in preparation) forms an technical platform for a fast and cost effective SNP and point mutation discovery.

**Claims**

1. A method for detecting a variation in a nucleic acid molecule said method comprising:

    (a) providing nucleic acids;
    (b) transforming the nucleic acids into one or more single stranded nucleic acid spatial conformer(s);
    (c) separating the conformer(s) under native conditions;
    (d) changing one condition of the separation at least one time during the separation wherein the change can impart a conformation change in the conformer(s);
    (e) detecting the mobility pattern of the conformer(s); and
    (f) comparing the mobility pattern of the conformer(s) to a mobility pattern of a control nucleic acid conformer so as to thereby detect a variation in the nucleic acid molecule,

    <u>wherein the condition is temperature,</u> and wherein the temperature is lowered during separation.

2. The method of claim 1, wherein the variation is a single base difference in said nucleic acid molecule compared to said control.

3. The method of claim 2, wherein said single base difference is a single nucleotide polymorphism.

4. The method of claim 2, wherein said single base difference is a mutation.

5. The method of claim 2, wherein said nucleic acid molecule comprises double stranded DNA.

6. The method of claim 1, wherein said nucleic acid molecule comprises RNA.

7. The method of claim 1, wherein said nucleic acids are obtained from a tissue sample or culture, cells, bacteria or viruses, transcribed in vitro from a DNA template or chemically synthesized.

8. The method of claim 1, wherein said nucleic acids are provided by PCR.

9. The method of claim 1, wherein said transformation of nucleic acids comprises subjecting said nucleic acids to physical, chemical or enzymatic means or a combination thereof, wherein the secondary structure of said nucleic acid is disrupted.

10. The method of claim 9, wherein said physical, chemical, or enzymatic means are selected from the group consisting of low pH, high pH, high temperature, low salt concentration, urea or formamide.

11. The method of claim 10, wherein said transformation comprises heating said nucleic acids to about 94°C.

12. The method of claim 1, wherein said separation step is selected from electrophoresis or chromatography.

13. The method of claim 12, wherein said separation step is polyacrylamide gel electrophoresis.

14. The method of claim 1, wherein the step of changing the condition during said separation changes the total energy of said single stranded nucleic acid spatial conformer.

15. The method of claim 1, wherein said nucleic acid molecule contains a fluorescent label.

16. The method of claim 1, wherein said nucleic acid molecule contains an electromagnetic label.

17. The method of claim 15, further comprising detection of said fluorescent label.

**18.** The method of claim 16, further comprising detection of said electromagnetic label.

**19.** The method of claim 1, wherein said detection is by silver staining.

**20.** The method of claim 1, wherein the control conformer is a wild-type sequence.

**21.** The method of claim 1, wherein the control conformer is a mutant sequence.

**22.** The method of claim 1, wherein the control conformer contains a sequence variation.

**23.** The method of claim 1, further comprising:

    (a) electro-eluting the nucleic acid conformer(s); and
    (b) determining the nucleic acid sequence of said conformer(s).

**Patentansprüche**

**1.** Verfahren zum Nachweis einer Variation in einem Nukleinsäuremolekül, wobei das Verfahren umfasst:

    a) Bereitstellen von Nukleinsäuren;
    b) Transformieren der Nukleinsäuren in ein oder mehrere einzelsträngige Nukleinsäureraumkonformer(e);
    c) Trennen des/der Konformer(e) unter nativen Bedingungen;
    d) Verändern einer Bedingung des Trennens mindestens ein Mal während der Trennung, wobei die Veränderung einen Konformationswechsel in dem/den Konformer(en) herbeiführen kann;
    e) Nachweisen des Mobilitätsmusters des/der Konformer(e); und
    f) Vergleichen des Mobilitätsmusters des/der Konformer(e) mit einem Mobilitätsmuster eines Kontroll-Nukleinsäurekonformers, um **dadurch** eine Variation in dem Nukleinsäuremolekül nachzuweisen,

wobei die Bedingung Temperatur ist und wobei die Temperatur während der Trennung verringert wird.

**2.** Verfahren gemäß Anspruch 1, wobei die Variation ein Einzelbasenunterschied in dem Nukleinsäuremolekül im Vergleich zu der Kontrolle ist.

**3.** Verfahren gemäß Anspruch 2, wobei der Einzelbasenunterschied ein Einzelnukleotidpolymorphismus ist.

**4.** Verfahren gemäß Anspruch 2, wobei der Einzelbasenunterschied eine Mutation ist.

**5.** Verfahren gemäß Anspruch 2, wobei das Nukleinsäuremolekül doppelsträngige DNA umfasst.

**6.** Verfahren gemäß Anspruch 1, wobei das Nukleinsäuremolekül RNA umfasst.

**7.** Verfahren gemäß Anspruch 1, wobei die Nukleinsäuren aus einer Gewebeprobe oder -kultur, Zellen, Bakterien oder Viren erhalten werden, in vitro aus einem DNA-Template transkribiert werden oder chemisch synthetisiert werden.

**8.** Verfahren gemäß Anspruch 1, wobei die Nukleinsäuren durch PCR bereitgestellt werden.

**9.** Verfahren gemäß Anspruch 1, wobei die Transformation der Nukleinsäuren das Unterwerfen der Nukleinsäuren gegenüber physikalischen, chemischen oder enzymatischen Mitteln oder einer Kombination davon umfassen, wobei die Sekundärstruktur der Nukleinsäure zerstört wird.

**10.** Verfahren gemäß Anspruch 9, wobei die physikalischen, chemischen oder enzymatischen Mittel ausgewählt sind aus der Gruppe, bestehend aus niedrigem pH-Wert, hohem pH-Wert, hoher Temperatur, niedriger Salzkonzentration, Harnstoff oder Formamid.

**11.** Verfahren gemäß Anspruch 10, wobei die Transformation das Erhitzen der Nukleinsäuren bis ungefähr 94°C umfasst.

**12.** Verfahren gemäß Anspruch 1, wobei der Trennungsschritt ausgewählt ist aus Elektrophorese oder Chromatographie.

**13.** Verfahren gemäß Anspruch 12, wobei der Trennungsschritt Polyacrylamidgelelektrophorese ist.

**14.** Verfahren gemäß Anspruch 1, wobei der Schritt des Veränderns der Bedingung während der Trennung die Gesamtenergie des einzelsträngigen Nukleinsäureraumkonformers verändert.

**15.** Verfahren gemäß Anspruch 1, wobei das Nukleinsäuremolekül eine Fluoreszenz-Markierung umfasst.

**16.** Verfahren gemäß Anspruch 1, wobei das Nukleinsäuremolekül eine elektromagnetische Markierung umfasst.

**17.** Verfahren gemäß Anspruch 15, weiterhin umfassend den Nachweis der Fluoreszenz-Markierung.

**18.** Verfahren gemäß Anspruch 16, weiterhin umfassend den Nachweis der elektromagnetischen Markierung.

**19.** Verfahren gemäß Anspruch 1, wobei der Nachweis erfolgt durch Silberfärbung.

**20.** Verfahren gemäß Anspruch 1, wobei das Kontrollkonformer eine Wildtypsequenz ist.

**21.** Verfahren gemäß Anspruch 1, wobei das Kontrollkonformer eine Mutantensequenz ist.

**22.** Verfahren gemäß Anspruch 1, wobei das Kontrollkonformer eine Sequenzvariation umfasst.

**23.** Verfahren gemäß Anspruch 1, weiterhin umfassend:

a) Elektroeluieren des/der Nukleinsäurekonformer(e); und
b) Nachweisen der Nukleinsäuresequenz des/der Konformer(e).

**Revendications**

**1.** Procédé de détection d'une variation dans une molécule d'acide nucléique, ledit procédé comprenant :

(a) la mise à disposition d'acides nucléiques ;
(b) la transformation des acides nucléiques en un ou plusieurs conformères spatiaux d'acides nucléiques simple brin ;
(c) la séparation du ou des conformères dans des conditions natives ;
(d) le changement d'une condition de la séparation au moins une fois pendant la séparation, le changement pouvant conférer un changement de conformation dans le ou les conformères ;
(e) la détection du schéma de mobilité du ou des conformères ; et
(f) la comparaison du schéma de mobilité du ou des conformères avec un schéma de mobilité d'un conformère d'acide nucléique témoin de façon à détecter de ce fait une variation dans la molécule d'acide nucléique,

procédé dans lequel la condition est la température, la température étant abaissée pendant la séparation.

**2.** Procédé selon la revendication 1, dans lequel la variation est une différence d'une seule base dans ladite molécule d'acide nucléique, par comparaison audit témoin.

**3.** Procédé selon la revendication 2, dans lequel ladite différence d'une seule base est un polymorphisme nucléotidique.

**4.** Procédé selon la revendication 2, dans lequel ladite différence d'une seule base est une mutation.

**5.** Procédé selon la revendication 2, dans lequel ladite molécule d'acide nucléique comprend un ADN double brin.

**6.** Procédé selon la revendication 1, dans lequel ladite molécule d'acide nucléique comprend un ARN.

**7.** Procédé selon la revendication 1, dans lequel lesdits acides nucléiques sont obtenus à partir d'un échantillon ou d'une culture de tissu, de cellules, de bactéries ou de virus, transcrits in vitro à partir d'une matrice d'ADN ou chimiquement synthétisés.

**8.** Procédé selon la revendication 1, dans lequel lesdits acides nucléiques sont mis à disposition par une PCR.

**9.** Procédé selon la revendication 1, dans lequel ladite transformation d'acides nucléiques consiste à soumettre lesdits acides nucléiques à des moyens physiques, chimiques ou enzymatiques, ou une combinaison de ces derniers, avec rupture de la structure secondaire dudit acide nucléique.

**10.** Procédé selon la revendication 9, dans lequel lesdits moyens physiques, chimiques ou enzymatiques sont choisis dans le groupe consistant en un faible pH, un pH élevé, une température élevée, une faible concentration de sels, l'urée ou le formamide.

**11.** Procédé selon la revendication 10, dans lequel ladite transformation consiste à chauffer lesdits acides nucléiques à environ 94°C.

**12.** Procédé selon la revendication 1, dans lequel ladite étape de séparation est choisie parmi l'électrophorèse ou la chromatographie.

**13.** Procédé selon la revendication 12, dans lequel ladite étape de séparation est une électrophorèse sur gel de polyacrylamide.

**14.** Procédé selon la revendication 1, dans lequel l'étape de changement de la condition au cours de ladite séparation modifie l'énergie totale dudit conformère spatial d'acide nucléique simple brin.

**15.** Procédé selon la revendication 1, dans lequel ladite molécule d'acide nucléique contient un marqueur fluorescent.

**16.** Procédé selon la revendication 1, dans lequel ladite molécule d'acide nucléique contient un marqueur électromagnétique.

**17.** Procédé selon la revendication 15, qui comprend en outre la détection dudit marqueur fluorescent.

**18.** Procédé selon la revendication 16, qui comprend en outre la détection dudit marqueur électromagnétique.

**19.** Procédé selon la revendication 1, dans lequel ladite détection s'effectue par coloration à l'argent.

**20.** Procédé selon la revendication 1, dans lequel le conformère témoin est une séquence de type sauvage.

**21.** Procédé selon la revendication 1, dans lequel le conformère témoin est une séquence mutante.

**22.** Procédé selon la revendication 1, dans lequel le conformère témoin contient une variation de séquence.

**23.** Procédé selon la revendication 1, qui comprend en outre :

(a) l'électro-élution du ou des conformères d'acides nucléiques ; et
(b) la détermination de la séquence d'acides nucléiques dudit ou desdits conformères.

# Fig.1

Fig.2

Fig.3

A

SSCP at 34°C

B

SSCP at 22°C

C

SSCP at 10°C

D

MSSCP at
34°C/22°C/10°C

## REFERENCES CITED IN THE DESCRIPTION

### Patent documents cited in the description

- US 4683195 A
- US 4683202 A, Mullis
- US 5719028 A
- US 5582989 A
- US 5633134 A, Shuber
- US 5858659 A, Sapolsky
- WO 0061805 A, Nerenberger, M
- WO 0050869 A, Arnold, L.
- WO 0020853 A, Liu
- WO 9812355 A, Monforte
- WO 9814616 A, Turano
- WO 02063043 A
- PL 0100012 W

### Non-patent literature cited in the description

- **Urdea et al.** *Gene,* 1987, vol. 61, 253-264
- **Barany.** *Proc.Natl.Acad.Sci.,* 1991, vol. 88, 189
- **Guatelli et al.** *Proc.Natl,Acad. Sci.,* 1990, vol. 87, 1874-1878
- *Electrophoresis,* vol. 20
- **Abrams et al.** *Genomics,* 1990, vol. 7, 463-475
- **Sheffield et al.** *Proc. Natl. Acad. Sci.,* 1989, vol. 86, 232-236
- **Lerman ; Silverstein.** *Meth. Enzymol.,* 1987, vol. 155, 482-501
- **Wartell et al.** *Nucl. Acids Res.,* 1990, vol. 18, 2699-2701
- **Orita et al.** *Genomics,* 1989, vol. 5, 874-879
- **SantaLucia, Jr.** *Proc. Natl. Acad. Sci. USA,* February 1998, vol. 95, 1460-1465
- **Kiyama, M. ; Fujita, T.** *Biotechniques,* 1996, vol. 21, 710-716
- **Collins, A. ; Lonjjou, C. ; Morton, N.** *Proc.Natl.Acad. Sci. USA,* 1999, vol. 96, 15173-15177
- **Ross et al.** *Anal Chem.,* 1997, vol. 15, 4197-202
- **Grace et al.** *Nucl. Acids Res.,* 1995, vol. 23, 4224-4226
- **Chen et al.** *Nucl. Acids Res.,* 1995, vol. 23, 4524
- **Rübben et al.** *Eur, J. Epidemiol.,* 1995, vol. 11, 301-306